(19) Europäisches Patentamt
European Patent Office
Office européen des brevets

(11) **EP 3 310 272 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(21) Anmeldenummer: **16736789.5**

(22) Anmeldetag: **09.06.2016**

(51) Int Cl.:
**A61B 17/16** (2006.01)

(86) Internationale Anmeldenummer:
**PCT/EP2016/000943**

(87) Internationale Veröffentlichungsnummer:
**WO 2016/202440 (22.12.2016 Gazette 2016/51)**

(54) **WERKZEUG FÜR DIE DILATATION VON KNOCHENBOHRUNGEN IN DER REKONSTRUKTIVEN CHIRURGIE UND WERKZEUGSATZ UMFASSEND EIN SOLCHES WERKZEUG**

TOOL FOR THE DILATATION OF DRILLED BONE HOLES IN RECONSTRUCTIVE SURGERY, AND TOOL SET COMPRISING SUCH A TOOL

OUTIL POUR LA DILATATION DE TROUS DANS L'OS LORS DE LA CHIRURGIE RECONSTRUCTIVE ET JEU D'OUTILS COMPRENANT UN TEL OUTIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.06.2015 DE 102015007541**

(43) Veröffentlichungstag der Anmeldung:
**25.04.2018 Patentblatt 2018/17**

(73) Patentinhaber:
• **REINHARD Feinmechanik GmbH**
**63128 Dietzenbach (DE)**
• **Missalla, Adalbert**
**61440 Oberursel (DE)**

(72) Erfinder:
• **MISSALLA, Adalbert**
**61440 Oberursel (DE)**
• **REINHARD, Helmut**
**60386 Frankfurt (DE)**

(74) Vertreter: **Oppermann, Mark**
**Oppermann & Oppermann**
**Patentanwälte**
**Am Wiesengrund 35**
**D-63075 Offenbach (DE)**

(56) Entgegenhaltungen:
US-A1- 2010 249 930   US-A1- 2012 059 469
US-A1- 2013 046 353   US-A1- 2013 096 677

## Beschreibung

TECHNISCHES GEBIET

Die vorliegende Erfindung bezieht sich auf eine Kombination aus einem Werkzeug für die Dilatation von Knochenbohrungen in der rekonstruktiven Chirurgie, einem Sehnenimplantat und einem Knochenimplantat gemäß dem Oberbegriff des Patentanspruchs 1. Derartige Werkzeuge kommen in der rekonstruktiven Chirurgie insbesondere bei der autologen (körpereigenen) Wiederherstellung von Kreuzbändern zur Ausbildung eines Presssitz-Implantatlagers im Femur massenweise zum Einsatz. Die Erfindung bezieht sich ferner auf einen Werkzeugsatz, der solche Werkzeuge umfasst.

STAND DER TECHNIK

Als Bänder bezeichnet man in der Anatomie faserartige, zumeist wenig dehnbare Bindegewebsstränge, welche die Aufgabe haben, die beweglichen Teile des Knochenskeletts so miteinander zu verbinden, dass die Beweglichkeit der Gelenke auf ein funktionell sinnvolles Maß beschränkt wird. Bei einer über dieses Maß hinausgehenden Dehnung, zum Beispiel in Folge einer Überlastung, kommt es häufig zu Bänderrissen, insbesondere am Kniegelenk. Um die Funktion des Gelenks wieder herzustellen, kann das gerissene Band operativ durch ein künstliches oder autologes Implantat ersetzt werden.

Bei der autologen Wiederherstellung des vorderen Kreuzbandes des Kniegelenks nach der sogenannten "All-Press-Fit"-Technik - die als fremdkörperfreie Operationsmethode mit biologischer und gelenknaher Einheilung des Sehnenimplantats gegenüber konventionellen Verfahren mit künstlichen, d.h. körperfremden Befestigungsmitteln besonders vorteilhaft ist, schon weil Abstoßreaktionen vermieden werden - wird üblicherweise kurzgefasst wie folgt vorgegangen:
In einem ersten Operationsschritt wird dem Patienten Sehnenmaterial (von der Semitendinosus und ggf. zusätzlich der Gracilissehne) entnommen, welches später zu einem Kreuzband-Sehnenimplantat weiterverarbeitet wird. Die Fixierung des Sehnenimplantats erfolgt, wie nachfolgend näher beschrieben wird, doppelt femoral und tibial über je eine Knochenbrücke und zusätzlich mit kortikospongiösen Knochenzylindern. Diese Knochenzylinder werden aus femoralen und tibialen Knochenbohrungen gewonnen.

Genauer gesagt werden eine durchgehende obere tibiale Knochenbohrung und eine femorale Knochenbohrung in der Art eines Sacklochs sowie eine weitere untere tibiale Knochenbohrung, ebenfalls in der Art eines Sacklochs, erzeugt. Die beiden tibialen Knochenbohrungen werden zur Erstellung der tibialen Knochenbrücke mittels eines kleinen Verbindungskanals verbunden. Am proximalen Ende der femoralen Knochenbohrung werden zwei kleine, die femorale Knochenbohrung parallel zu deren Mittelachse verlängernde und durchgehende Fadenkanäle für die femorale Knochenbrücke erzeugt. Die Erstellung der drei erstgenannten Knochenbohrungen erfolgt mit Hilfe von z.B. Diamanthohlschleifen oder anderen geeigneten hohlzylindrischen Werkzeugen, wobei die gewonnenen Bohrkerne (Knochenzylinder) später zur Verankerung des Sehnenimplantats in den Knochenbohrungen und zum Abschluss der tibialen Knochenbohrungen verwendet werden.

Zur Erstellung des ca. 7 cm langen Sehnenimplantats wird das entnommene Sehnenmaterial - nach Bestimmung der Sehnenstärke mit Hilfe einer Lehre, mittels der ein äquivalenter Sehnendurchmesser ermittelt wird - an den Enden aneinandergenäht, zu einer Schlaufe umgeschlagen und dadurch doppelt verwendet. Proximal wird das Sehnenimplantat von zwei Fäden gefasst; distal wird der aus der unteren Knochenbohrung der Tibia gewonnene, ca. 15 mm lange Knochenzylinder eingenäht. Das Sehnenimplantat wird hierbei standardisiert mit proximalen und distalen Fäden zur Sicherung über den Knochenbrücken auf einem speziell für diese Technik entwickelten Arbeitsbrett vorbereitet, das in der Druckschrift DE 20 2014 007 804 U1 näher beschrieben ist.

Da die Stärke, d.h. der Querschnitt des verwendeten Sehnenmaterials von Patient zu Patient unterschiedlich groß ist, während die Knochenzylinder mit standardisierten Werkzeugen, z.B. den vorerwähnten Diamanthohlschleifen gewonnen werden und somit definierte Durchmesser besitzen, ist das vorbereitete Sehnenimplantat an seinen in den Knochenbohrungen aufzunehmenden Enden im Querschnitt individuell verschieden groß. Auch deshalb ist die femorale wie auch die obere tibiale Knochenbohrung zusätzlich aufzuweiten.

Hierfür kommen als stabförmige Treibwerkzeuge ausgebildete Dilatatoren zum Einsatz, die einen Schaft mit einem ersten Ende und einem zweiten Ende aufweisen, wobei das erste Ende einen bezüglich der Mittelachse des Dilatators symmetrischen oder asymmetrischen Aufweitungsquerschnitt besitzt. Dilatatoren mit einem asymmetrischen Aufweitungsquerschnitt werden insbesondere bei großen Sehnenstärken eingesetzt, um ein im Querschnitt gesehen tangential an die Knochenbohrung anschließendes Sehnenlager auszubilden.

Die Aufweitung der Knochenbohrungen erfolgt in mehreren, z.B. bis zu 8 Dilatationsstufen pro Knochenbohrung, um eine Überlastung des Knochens zu vermeiden. Mit von Dilatator zu Dilatator zunehmendem Aufweitungsquerschnitt werden die Dilatatoren durch Aufbringung äußerer Kräfte am zweiten Ende des Schafts mittels eines Hammers in die jeweilige Knochenbohrung eingeschlagen und anschließend wieder unter Aufbringung einer entgegengesetzten äußeren Kraft am zweiten Ende des Schafts aus der entsprechenden Knochenbohrung herausgetrieben. Die Kraft zum Austreiben des Dilatators wird dabei durch eine um den Schaft des Dilatators gelegte Impulsmasse aufgebracht, die gegen eine Anschlagfläche am zweiten Ende des Dilatators von der Knochenbohrung weg beschleunigt wird.

Nach Ausbildung der Implantatlager wird das vorbereitete Sehnenimplantat mittels der proximalen Fäden, welche vorher durch die die femorale Knochenbohrung verlängernden Fadenkanäle hindurchgezogen wurden, durch die geeignet aufgeweitete, obere tibiale Knochenbohrung bis vor die geeignet aufgeweitete, femorale Knochenbohrung eingezogen. Vor dem kompletten Einzug in die femorale Knochenbohrung wird das Sehnenimplantat an dem in der oberen tibialen Knochenbohrung aufzunehmenden Ende um 90° lateral gedreht. Dadurch werden die Implantatanteile so platziert, dass ein anteromediales und ein posterolaterales Bündel entstehen, wodurch die anatomische Form des natürlichen Kreuzbandes nachgebildet wird. Das Sehnenimplantat wird nun 15 mm tief in die femorale Knochenbohrung eingezogen (und dabei teilweise tibial eingeschlagen) und sodann von oberhalb des Femurs mittels der proximalen Fäden gespannt, worauf der aus der Knochenbohrung des Femurs gewonnene, geeignet gekürzte Knochenzylinder mittels eines Applikators - umfassend ebenfalls ein stabförmiges Treibwerkzeug und eine Führung für den Knochenzylinder - in die aufgeweitete femorale Knochenbohrung zur Erzeugung der "Press-Fit"-Verbindung eingetrieben und somit fixiert wird.

Nach Streckung des Kniegelenks erfolgt die an die individuelle Geometrie des Gelenks selbstadaptierende Anpassung der Kreuzbandspannung. Die unteren der distalen Fäden des Implantats werden von der oberen tibialen Knochenbohrung durch den senkrechten Verbindungskanal in die untere tibiale Knochenbohrung gezogen und sodann mit den oberen der distalen Fäden aus der oberen tibialen Knochenbohrung verknotet (Knochenbrücke). Abschließend werden die Knochenbohrungen in der Tibia mit Abschnitten des aus der oberen Knochenbohrung der Tibia gewonnenen Knochenzylinders verblockt, worauf die beiden proximalen Fäden des Implantats über der femoralen Knochenbrücke verknotet werden.

Die wie vorbeschrieben erzeugte femorale "Press-Fit"-Verbindung dient - neben einer Verbesserung des Einheilprozesses infolge einer weitgehenden Vermeidung von Spalten zwischen dem Sehnenimplantat und dem dieses aufnehmenden Implantatlager im Knochen - insbesondere dazu, das Sehnenimplantat vor seiner Einheilung ohne zusätzliche Fixierung durch Nahtmaterial möglichst auszugsfest in dem Implantatlager zu befestigen. Die hierbei erreichte Auszugsfestigkeit, auch.Primärstabilität genannt, wird durch die im Implantatlager am Sehnenimplantat wirkenden Kräfte bestimmt, welche wiederum von der Geometrie des Implantatlagers, des Knochenzylinders und des Sehnenimplantats sowie den intrinsischen Eigenschaften des Sehnenmaterials und des anisotropen Knochenmaterials abhängig sind.

Der Operateur findet während des operativen Eingriffs also nie die gleichen Voraussetzungen vor und muss mit dem Ziel, dem Patienten postoperativ eine möglichst hohe Primärstabilität der Verbindung zu verschaffen, die "Press-Fit"-Konditionierung vornehmen, was ein hohes Maß an Fallerfahrung erfordert. So ist es nicht verwunderlich, dass es in der Praxis in Folge fehlender Standards zu einer erheblichen Streuung bei der erreichten Primärstabilität und damit dem Erfolg bzw. Misserfolg solcher Eingriffe kommt. Dies steht freilich einer breiten Anwendung der "All-Press-Fit"-Technik entgegen. Wünschenswert wäre eine standardisierte Technologie für diese Eingriffe, die die Grundlage für eine breite Vorteilsnahme durch Patienten und Kostenträger bilden würde.

Das Dokument US 2010/249930 A1 offenbart eine Kombination aus einem Werkzeug, einem Sehnenimplantat und einem Knochenimplantat gemäß dem Oberbegriff des Anspruchs 1.

## AUFGABENSTELLUNG

Der Erfindung liegt vor diesem Hintergrund die Aufgabe zugrunde, ein Werkzeug für insbesondere die Dilatation von Knochenbohrungen zur Ausbildung eines Implantatlagers für ein Implantat in der rekonstruktiven Chirurgie bzw. einen entsprechenden Werkzeugsatz zu schaffen, das/der es ermöglicht, weitgehend unabhängig von den patientenindividuellen Voraussetzungen am Ort des Eingriffs zu reproduzierbar guten Operationsergebnissen, insbesondere einer möglichst hohen Primärstabilität der Verbindung des Implantats im Implantatlager zu gelangen.

## DARSTELLUNG DER ERFINDUNG

Diese Aufgabe wird durch die in den Patentansprüchen 1, 11, 12 bzw. 14 angegebenen Merkmale gelöst. Vorteilhafte und/oder zweckmäßige Weiterbildungen der Erfindung sind Gegenstand der Patentansprüche 2 bis 10, 13 und 15.

Bei einem Werkzeug für die Dilatation von Knochenbohrungen in der rekonstruktiven Chirurgie, mit einem Schaft, der ein erstes Ende mit einem Aufweitungsabschnitt hat, welcher eine definierte Querschnittsfläche ($A_B$) besitzt und mittels dessen die Knochenbohrung aufweitbar ist, und ein zweites Ende aufweist, über das eine Kraft einleitbar ist, um das Werkzeug in die Knochenbohrung einzutreiben oder aus dieser auszutreiben, wobei die Knochenbohrung dazu dient, ein Sehnenimplantat mit einer Querschnittsfläche ($A_S$) und ein Knochenimplantat mit einer Querschnittsfläche ($A_K$) in einem Presssitz aufzunehmen; bemisst sich erfindungsgemäß die Querschnittsfläche ($A_B$) des Aufweitungsabschnitts nach folgender Beziehung:

$$A_B = \frac{(A_S + A_K)}{(1 + \alpha)}$$

mit $\alpha$: einem Kompressionsfaktor, der in einem Bereich von 0,30 bis 0,60, vorzugsweise in einem Bereich von 0,32 bis 0,57, mehr bevorzugt in einem Bereich von 0,38 bis 0,5 liegt und idealerweise etwa 0,44 beträgt.

Nach dem Grundgedanken der Erfindung wird also über den Kompressionsfaktor ($\alpha$) ein Verhältnis der aufsummierten Querschnittsflächen ($A_S$, $A_K$) der "freien", d.h. noch nicht im auszubildenden, zugeordneten Implantatlager aufgenommenen Sehnen- und Knochenimplantate zu der Querschnittsfläche ($A_B$) des Aufweitungsabschnitts des Dilatators, die im Wesentlichen der Querschnittsfläche des Implantatlagers entspricht, definiert. Mit Durchführung einer "Press-Fit"-Fixation unter Berücksichtigung des Kompressionsfaktors ($\alpha$) im beanspruchten Bereich bzw. mit der beanspruchten Größe, den/die die Erfinder im Rahmen von wissenschaftlichen Untersuchungen gefunden und in weiteren Versuchen verifiziert haben, lässt sich bei üblichen - in der Größe ohnehin materialmäßig nach "unten" und anatomisch nach "oben" begrenzten - Einbindungstiefen des (Gesamt)Implantats im Implantatlager eine hohe Primärstabilität der Verbindung des Implantats im Implantatlager erreichen. Dadurch, dass erfindungsgemäß die für eine gute Primärstabilität erforderliche "Press-Fit"-Konditionierung auf wenige Querschnittsgrößen zurückgeführt wird, die über den Kompressionsfaktor ($\alpha$) in eine definierte Relation zueinander gesetzt werden, ergibt sich sowohl für einen Hersteller der Dilatationswerkzeuge als auch für einen Operateur eine einfache und leicht zu handhabende Handlungsanweisung: Für vorgegebene Querschnittsflächen ($A_S$, $A_K$) des Sehnenimplantats und des Knochenimplantats kann die aufweitungswirksame Querschnittsfläche ($A_B$) des Dilatationswerkzeugs unter Berücksichtigung des Kompressionsfaktors ($\alpha$) problemlos bemessen bzw. für jeden Patienten individuell ausgewählt werden. Durch die somit vorgeschlagene Standardisierung der Dilatationswerkzeuge in Abhängigkeit von vorbestimmten Größen ist es insbesondere auch für Operateure ohne große Fallerfahrung leichter möglich, die "All-Press-Fit"-Technik prozesssicher und erfolgreich anzuwenden.

Die Geometrie der Querschnittsfläche des Aufweitungsabschnitts des Dilatators kann den jeweiligen Erfordernissen entsprechend frei gewählt werden - z.B. im Wesentlichen dreieckig ausgebildet sein - und demgemäß Symmetrien aufweisen oder auch nicht, solange das obige Verhältnis der Querschnittsflächen unter Berücksichtigung des Kompressionsfaktors eingehalten wird. In einer ersten, besonders einfachen Variante des Werkzeugs kann dieses derart gestaltet sein, dass der Aufweitungsabschnitt des ersten Endes des Schafts für eine symmetrische Dilatation der Knochenbohrung einen kreisförmigen Querschnitt mit einem Durchmesser ($d_D$) aufweist. Aufgrund der hiermit bewirkten symmetrischen Aufweitung der Knochenbohrung, bei der das angrenzende Knochenmaterial über dem Umfang des Aufweitungsabschnitts gesehen gleichmäßig radial nach außen verdrängt wird, ist diese Art der Aufweitung, die vornehmlich bei kleinen Sehnenimplantatdurchmessern eingesetzt wird, besonders schonend für den Knochen. Vor dem Hintergrund der vorbeschriebenen Querschnittsrelation zwischen Implantat und Implantatlager unter Berücksichtigung des Kompressionsfaktors ($\alpha$) ist hierbei der Durchmesser ($d_D$) des Aufweitungsabschnitts nach folgender Beziehung ermittelbar:

$$d_D = \sqrt{d_B{}^2 + \frac{d_S^2 + d_K^2 - d_B^2(1 + \alpha)}{(1 + \alpha)}}$$

mit $d_B$ : einem Durchmesser der Knochenbohrung (vor der Aufweitung),
$d_K$ : einem Durchmesser des Knochenimplantats (das wie eingangs beschrieben als Knochenzylinder mittels eines hohlzylindrischen Werkzeugs, z.B. einer Diamanthohlschleife gewonnen werden kann),
$d_S$ : einem äquivalenten Durchmesser des Sehnenimplantats (der wie oben schon erwähnt mittels einer Lehre bestimmbar ist, die eine Mehrzahl von im Durchmesser definiert gestuften zylindrischen Bohrungen aufweist) und
$\alpha$ : dem vorerwähnten Kompressionsfaktor.

Da sich im Falle der Verwendung eines hohlzylindrischen Werkzeugs zur gleichzeitigen Erzeugung von Knochenbohrung und Knochenzylinder die Durchmesser der Knochenbohrung und des Knochenimplantats ohne Weiteres aus der Geometrie des hohlzylindrischen Werkzeugs ergeben, verbleibt als zu bestimmende Variable der äquivalente Durchmesser des Sehnenimplantats. Dieser entspricht dem Durchmesser derjenigen Bohrung der Sehnenlehre, durch welche das Sehnenimplantat mit im Verhältnis geringen Kräften hindurchgezogen werden kann, um lediglich eine dieser Bohrung entsprechende zylindrische, weitestgehend hohlraumfreie Form zu erhalten, ohne jedoch dabei im eigentlichen Sehnenmaterial komprimiert zu werden.

In einer zweiten Variante des Werkzeugs kann die Querschnittform des Aufweitungsabschnitts des ersten Endes des Schafts für eine asymmetrische Dilatation ausgehend von einem bezüglich einer Mittelachse des Schafts zentrierten, kreisförmigen Grundquerschnitt auf einer Seite oder auf bezüglich der Mittelachse diametral gegenüberliegenden Seiten erhaben ausgebildet sein. Somit wird die Knochenbohrung beim Einschlagen des Dilatators im Querschnitt gesehen asymmetrisch ausgeformt, um zu einer oder beiden Seiten der Knochenbohrung ein Sehnenlager auszubilden, in welches das nur begrenzt verformbare Sehnenimplantat beim Einschlagen des Knochenimplantats ausweichen kann. Hierdurch wird insbesondere ein übermäßiges Verquetschen eines im Verhältnis querschnittsgroßen Sehnenimplantats verhindert; auch wird auf diese Weise - verglichen mit symmetrisch ausgebildeten "Press-Fit"-Verbindungen - bei querschnittsgrößeren Sehnenimplantaten die Möglichkeit der Bildung von den Einheilprozess verschlechternden Spalten oder Hohlräumen weiter reduziert.

Bei der zweiten Variante des Werkzeugs besitzt der Aufweitungsabschnitt des ersten Endes des Schafts vorzugsweise einen ovalen Querschnitt ($A_B$), dessen Umfangskontur durch zwei Halbkreise gleichen Durchmessers ($d_B$) und zwei die Halbkreise verbindenden Geraden gleicher Länge (e) gebildet ist, wodurch das vorerwähnte Sehnenlager knochenmaterialschonend ohne scharfe Übergänge oder Kanten tangential an die Knochenbohrung anschließend ausgebildet werden kann. Wiederum vor dem Hintergrund des vorbeschriebenen Querschnittsverhältnisses zwischen Implantat und Implantatlager unter Berücksichtigung des Kompressionsfaktors ($\alpha$) ist dabei die Länge (e) nach der folgenden Beziehung ermittelbar:

$$e = \frac{1}{d_B} \frac{\pi}{4} \frac{[d_S^2 + d_K^2 - d_B^2(1+\alpha)]}{(1+\alpha)}$$

mit

$d_B$: einem Durchmesser der Knochenbohrung,
$d_K$: einem Durchmesser des Knochenimplantats,
$d_S$: einem äquivalenten Durchmesser des Sehnenimplantats und
$\alpha$: dem vorerwähnten Kompressionsfaktor.

Im weiteren Verfolg des Erfindungsgedankens kann sich zwischen einem Endabschnitt und dem Aufweitungsabschnitt des ersten Endes des Schafts des Werkzeugs ein Übergangsabschnitt erstrecken, dessen Querschnitt sich von einem größten Querschnitt des Endabschnitts zu dem Querschnitt ($A_B$) des Aufweitungsabschnitts kontinuierlich erweitert, was wiederum einem knochenmaterialschonenden Aufweiten der Knochenbohrung förderlich ist. Über eine geeignete Wahl der Form der Querschnittserweiterung des Übergangsabschnitts kann hierbei auf den Fortschritt der Aufweitung und die Höhe der örtlichen Aufweitungskräfte in radialer und axialer Richtung in Abhängigkeit vom Werkzeugvortrieb gezielt Einfluss genommen werden. So kann den jeweiligen Erfordernissen entsprechend eine Außenkontur des Übergangsabschnitts im Bereich der Querschnittserweiterung von der Seite gesehen z.B. linear, S-förmig oder in der Form einer Logarithmusfunktion ausgebildet sein, während der Übergangsabschnitt im Querschnitt gesehen z.B. tropfenförmig oder oval geformt sein kann.

Grundsätzlich ist es möglich, das Werkzeug an seinem ersten Ende mit z.B. einem kegelstumpfförmigen Endabschnitt oder auch nur einer endseitigen Fase zu versehen. In einer bevorzugten Ausgestaltung weist das erste Ende des Schafts indes einen kugelkalottenförmigen Endabschnitt auf. Ein solcher Endabschnitt erleichtert in vorteilhafter Weise insbesondere ein bezüglich der Knochenbohrung zentriertes Ausrichten des Werkzeugs.

Prinzipiell ist es möglich, im Schaft am zweiten Ende desselben einen Längsschlitz mit einer dem ersten Ende zugewandten Einleitungsfläche auszubilden, gegen die z.B. die eingangs erwähnte Impulsmasse mit einer Gegenfläche beschleunigt werden kann, um am Werkzeug die äußeren Kräfte in Richtung weg vom ersten Ende aufzubringen und damit das Werkzeug aus der Knochenbohrung auszutreiben. Im Hinblick auf eine demgegenüber geringere Komplexität, stabilere Ausgestaltung und bessere Reinigungsmöglichkeit ist es indes bevorzugt, wenn das zweite Ende des Schafts zur Einleitung der axialen Austreibkräfte mit einer die Krafteinleitungsfläche ausbildenden Querschnittserweiterung versehen ist.

Hierbei weist die Querschnittserweiterung auf ihrer dem ersten Ende des Schafts zugewandten Seite vorzugsweise eine ballig ausgestaltete Ringfläche auf. Gegenüber ebenfalls denkbaren Ausgestaltungen, wie etwa einem Anschlagstift oder einzelnen Vorsprüngen, die symmetrisch oder unsymmetrisch am Umfang des Schafts verteilt angeordnet sein können, hat das Vorsehen einer Ringfläche insbesondere den Vorteil, dass es aufgrund der im Verhältnis großen umlaufenden Kontaktfläche bei der Kraftübertragung zu einer nur geringen Flächenpressung kommt, was die Gefahr einer Beschädigung reduziert. Darüber hinaus ist die ballige Ausgestaltung der Ringfläche insbesondere im Hinblick auf die Vermeidung von Querkräften von Vorteil; die äußeren Kräfte werden auch bei einer Winkelfehlstellung einer in Längsrichtung des Schafts beschleunigten Impulsmasse mit deren Anschlag an der Ringfläche in einer bezüglich der Längsachse des Schafts im Wesentlichen axialen Richtung eingeleitet.

In einer besonders einfachen Ausgestaltung kann das Werkzeug am zweiten Ende des Schafts eine Kraftübertragungsfläche zur Einleitung der äußeren Kräfte in Richtung des ersten Endes des Schafts, d.h. zum Eintreiben des Werkzeugs aufweisen, die senkrecht zu der Längsachse des Schafts steht, so dass diese die Flächennormale der Kraftübertragungsfläche bildet. Hierbei können beim Eintreiben des Werkzeugs mittels eines Hammers im Falle einer außermittigen Krafteinleitung infolge eines exzentrischen Auftreffens des Hammers auf der Kraftübertragungsfläche des Werkzeugs aber unerwünschte Querkräfte entstehen. In einer bevorzugten Ausgestaltung des Werkzeugs wird dem dadurch begegnet, dass das zweite Ende des Schafts zur Einleitung der axialen Eintreibkräfte einen kugelkalottenförmigen Endabschnitt aufweist. Ein vornehmlich in einer Richtung entlang des Schafts zu dessen ersten Ende hin beschleunigter Hammer trifft den Schaft mit seiner Kraftübertragungsfläche stets an der "vorstehenden" Kugelkalotte und somit im Bereich der Längsachse des Schafts.

In zweckmäßiger Ausgestaltung bilden mehrere, d.h. wenigstens zwei Werkzeuge einen Werkzeugsatz für eine stufenweise Dilatation einer Knochenbohrung als Presssitz-Implantatlager für ein kombiniertes Sehnen- und Knochenimplantat, wobei die Werkzeuge sich hinsichtlich ihres aufweitungswirksamen Querschnitts unterscheiden, um die erforderliche Gesamtaufweitung knochenmaterialschonend auf mehrere Schritte zu verteilen. Hierbei ist in einer ersten Variante des Werkzeugsatzes das querschnittsgrößte Werkzeug zur Ausbildung der finalen Geometrie des Implantatlagers so ausgebildet, dass sich das vorbeschriebene Querschnittsverhältnis zwischen Implantat und Implantatlager unter Berücksichtigung des Kompressionsfaktors ($\alpha$) ergibt. Durch die stufenweise Dilatation muss für die Operation unabhängig vom Durchmesser des Sehnenimplantats auch nur ein Bohrwerkzeug mit einem vorbestimmten Durchmesser zur Erstellung der aufzuweitenden Knochenbohrung vorgehalten werden.

In einer zweiten Variante eines solchen Werkzeugsatzes unterscheiden sich die Werkzeuge ebenfalls hinsichtlich ihres aufweitungswirksamen Querschnitts, sind dabei allerdings so im Querschnitt gestuft, dass jedes Werkzeug für einen bestimmten Sehnenquerschnitt ($A_S$) nach Maßgabe des vorbeschriebenen Querschnittsverhältnisses zwischen Implantat und Implantatlager unter Berücksichtigung des Kompressionsfaktors ($\alpha$) zur Ausbildung der finalen Geometrie des zu dem jeweiligen Sehnenquerschnitt ($A_S$) gehörigen Implantatlagers ausgebildet ist. Während bei der ersten Variante des Werkzeugsatzes für jeden Sehnenquerschnitt ($A_S$) ein hierauf "zugeschnittener" Werkzeugsatz vorzuhalten ist, was für bestimmte Anwendungsfälle ausreichend sein mag, kann der Werkzeugsatz gemäß der zweiten Variante universell für die verschiedenen vorzufindenden Sehnenquerschnitte ($A_S$) eingesetzt werden. Dies vereinfacht die Vorhaltung der für die Operationen benötigten Werkzeuge und hat darüber hinaus infolge der erzielten Werkzeugreduktion insbesondere auch Kostenvorteile.

Bei diesen Werkzeugsätzen können die Werkzeuge des jeweiligen Werkzeugsatzes so ausgestaltet sein, dass jedes Werkzeug am Aufweitungsabschnitt eine Umfangskontur mit definierter Länge besitzt, die sich von einem Werkzeug zum nächstgrößeren Werkzeug um einen Proportionalitätsfaktor ($\beta$) vergrößert, der in einem Bereich von 1,029 bis 1,039 liegt und vorzugsweise 1,035 beträgt. Die durch einen solchen Vergrößerungsfaktor vorgegebene und zugleich begrenzte Aufweitungsschrittweite bei der Dilatation hat sich im Hinblick auf einen zügigen Operationsfortschritt bei gleichzeitig kontrollierter Knochenbelastung im Rahmen der von den Erfindern durchgeführten Untersuchungen als vorteilhaft erwiesen.

Insoweit wurden erfindungsgemäße Dilatationswerkzeuge und Werkzeugsätze mit solchen Dilatationswerkzeugen beschrieben, die dazu dienen, eine vorher erzeugte Knochenbohrung zur weiteren Ausbildung eines Implantatlagers in definierter Weise aufzuweiten, um eine insbesondere im Hinblick auf eine hohe Primärstabilität der Verbindung zwischen dem Implantatlager und dem hierin aufgenommenen, kombinierten Sehnen- und Knochenimplantat möglichst gute "Press-Fit"-Konditionierung des Implantatlagers vorzunehmen. Der hiermit verbundene Grundgedanke der vorliegenden Erfindung ist indes auch auf andere Werkzeugkonzepte für die rekonstruktive Chirurgie übertragbar, bei denen das Werkzeug dazu dient, eine Aussparung in einem Knochen als Implantatlager insbesondere orginär zu erzeugen.

Bei einem solchen alternativen Werkzeugkonzept umfasst ein Werkzeug für die Erzeugung einer Aussparung in einem Knochen in der rekonstruktiven Chirurgie erfindungsgemäß einen ersten Werkzeugteil, welcher eine definierte Querschnittsfläche ($A_B$) besitzt und dessen Geometrie die Geometrie der erzeugten Aussparung im Knochen bestimmt, so dass die Querschnittsfläche der erzeugten Aussparung im Knochen der Querschnittsfläche ($A_B$) des ersten Werkzeugteils entspricht, und einen zweiten Werkzeugteil, über den das Werkzeug zur Erzeugung der Aussparung relativ zum Knochen zustellbar ist, wobei die Aussparung im Knochen dazu dient, ein Sehnenimplantat mit einer Querschnittsfläche ($A_S$) und ein Knochenimplantat mit einer Querschnittsfläche ($A_K$) in einem Presssitz aufzunehmen, und wobei die Querschnittsfläche ($A_B$) des ersten Werkzeugteils sich ebenfalls nach folgender Beziehung bemisst:

$$A_B = \frac{(A_S + A_K)}{(1 + \alpha)}$$

mit $\alpha$: einem Kompressionsfaktor, der in einem Bereich von 0,30 bis 0,60, vorzugsweise in einem Bereich von 0,32 bis 0,57, mehr bevorzugt in einem Bereich von 0,38 bis 0,5 liegt und idealerweise etwa 0,44 beträgt.

Ein solches Werkzeug kann beispielsweise als Bohr-, Fräs- oder Schleifwerkzeug mit geometrisch bestimmten bzw. unbestimmten Schneiden ausgebildet sein, wobei der erste Werkzeugteil z.B. hohlzylindrisch geformt ist und stirnseitig die Schneiden trägt, während über den zweiten Werkzeugteil sowohl die axiale Zustellung des Werkzeugs als auch dessen Drehantrieb erfolgt. Hierbei können die radiale Breite der Schneiden und die Wandstärke des hohlzylindrischen Werkzeugteils so dimensioniert werden, dass bei der Erzeugung der Aussparung im Knochen zugleich (a) innenumfangsseitig als (Bohr)Kern ein Knochenzylinder entsteht, der - ggf. nach geeigneter Kürzung - im Weiteren als Knochenimplantat hergenommen werden kann, und (b) außenumfangsseitig eine hierzu passende finale Geometrie des Implantatlagers mit optimierter "Press-Fit"-Konditionierung ausgebildet wird. Ein Werkzeugsatz mit solchen Werkzeugen könnte in Abhängigkeit von möglichen Querschnitten der einzusetzenden Sehnenimplantate Werkzeuge mit von Werkzeug zu Werkzeug gestuften Wandstärken und/oder Außendurchmessern des ersten Werkzeugteils umfassen.

In analoger Weise könnte bei einem solchen alternativen Werkzeugkonzept das Werkzeug als eine Art Stanzwerkzeug mit einer stirnseitigen Ringschneide an dem als Hohlkörper geformten ersten Werkzeugteil ausgebildet werden, während über den zweiten Werkzeugteil eine - ggf. hochfrequent oszillierende - axiale Zustellung des Werkzeugs erfolgt. Anders als bei rotierenden Werkzeugen könnten mit solchen Werkzeugen auch Implantatlager und Knochenimplantate unmittelbar erzeugt werden, deren Form von der Rotationsform abweicht.

Für den Fachmann ist schließlich ersichtlich, dass bei einem Werkzeugsatz mit erfindungsgemäßen Werkzeugen, deren als Hohlkörper geformte erste Werkzeugteile auch dazu dienen, das Knochenimplantat bzw. ein Rohteil hierfür zu generieren, die durch die Außenumfangskontur des bearbeitungswirksamen ersten Werkzeugteils definierte und auf die im Knochen erzeugte Aussparung übertragene Querschnittsfläche ($A_B$) konstant gehalten werden kann, während die Innenumfangskontur des bearbeitungswirksamen ersten Werkzeugteils, welche die Querschnittsfläche ($A_K$) des Knochenimplantats bestimmt, von Werkzeug zu Werkzeug variiert wird, um jeweils für einen bestimmten Querschnitt ($A_S$) eines in die Aussparung im Knochen einzusetzenden Sehnenimplantats unter Berücksichtigung des erfindungsgemäßen Kompressionsfaktors eine optimierte "Press-Fit"-Konditionierung vorzunehmen.

KURZE BESCHREIBUNG DER ZEICHNUNGEN

Im Folgenden wird die Erfindung anhand bevorzugter Ausführungsbeispiele unter Bezugnahme auf die beigefügten teilweise schematischen bzw. vereinfachten Zeichnungen näher erläutert, wobei gleiche oder entsprechende Teile mit den gleichen Bezugszeichen versehen sind. In den Zeichnungen zeigen:

Fig. 1 eine perspektivische Darstellung eines Werkzeugsatzes nach einem bevorzugten Ausführungsbeispiel der Erfindung, umfassend (wenigstens) zwei stabförmige Werkzeuge zur Dilatation von Knochenbohrungen, von denen eines - hier das obere - einen querschnittskleineren Aufweitungsabschnitt für eine symmetrische (Vor)Dilatation der Knochenbohrung hat, während das andere hier das untere - einen querschnittsgrößeren Aufweitungsabschnitt für eine asymmetrische (Fertig)Dilatation der Knochenbohrung besitzt;

Fig. 2 eine Prinzipskizze einer symmetrisch aufgeweiteten Knochenbohrung im Schnitt, in der ein Sehnenimplantat mittels eines zylindrischen Knochenimplantats zur axialen Fixierung verpresst ist ("Press-Fit"-Verbindung), wobei die Knochenbohrung vor der symmetrischen Dilatation durch eine gestrichelte Linie angedeutet ist;

Fig. 3 eine Draufsicht auf den für die symmetrische Aufweitung ausgebildeten Dilatator des Werkzeugsatzes gemäß Fig. 1;

Fig. 4 eine Schnittansicht des Dilatators zur symmetrischen Aufweitung gemäß Fig. 1 entsprechend der Schnittverlaufslinie IV-IV in Fig. 3;

Fig. 5 eine Prinzipskizze einer asymmetrisch, hier oval aufgeweiteten Knochenbohrung im Schnitt, in der ein Sehnenimplantat wiederum mittels eines zylindrischen Knochenimplantats zur axialen Fixierung verpresst ist ("Press-Fit"-Verbindung), wobei die Knochenbohrung vor der asymmetrischen Dilatation durch eine gestrichelte Linie angedeutet ist;

Fig. 6 eine Draufsicht auf den für die asymmetrische Aufweitung ausgebildeten Dilatator des Werkzeugsatzes gemäß Fig. 1;

Fig. 7 eine Schnittansicht des Dilatators zur asymmetrischen Aufweitung gemäß Fig. 1 entsprechend der Schnittverlaufslinie VII-VII in Fig. 6;

Fig. 8 eine Schnittansicht des Dilatators zur asymmetrischen Aufweitung gemäß Fig. 1 entsprechend der Schnittverlaufslinie VIII-VIII in Fig. 6; und

Fig. 9 eine im Maßstab gegenüber der Darstellung in Fig. 8 vergrößerte Prinzipskizze zum Querschnitt des Aufweitungsabschnitts des Dilatators zur asymmetrischen Aufweitung gemäß Fig. 1, der weitere geometrische Details des Aufweitungsquerschnitts zu entnehmen sind.

DETAILLIERTE BESCHREIBUNG DER AUSFÜHRUNGSBEISPIELE

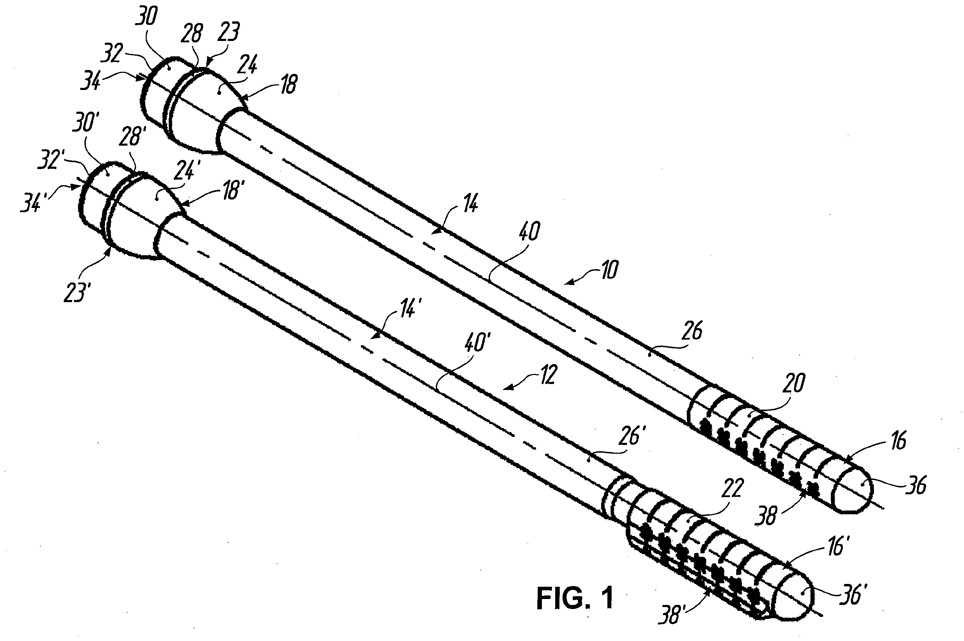

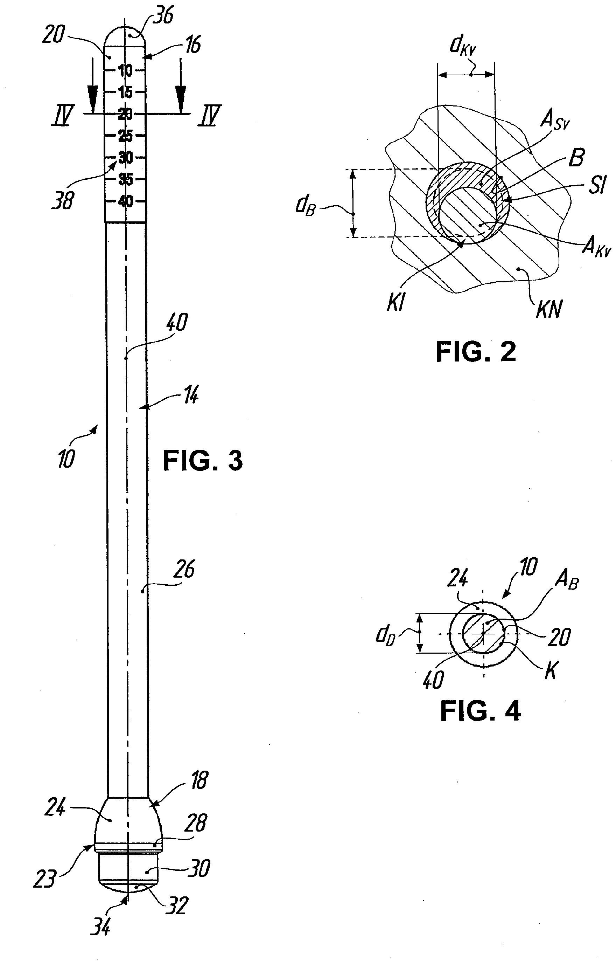

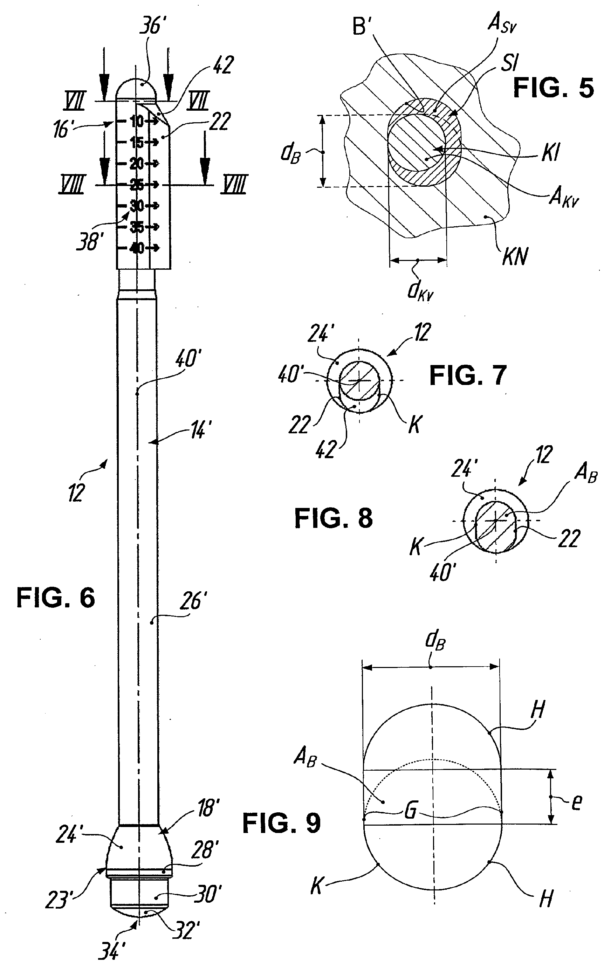

Die Fig. 1 zeigt exemplarisch einen chirurgischen Instrumentensatz mit zwei stabförmigen (Treib)Werkzeugen 10, 12 für die Dilatation von Bohrungen B, B' in einem Knochen KN in der rekonstruktiven Chirurgie, gemeinhin auch "Dilatatoren" genannt. Die Knochenbohrungen B, B' (vgl. die Fig. 2 und 5) dienen hierbei dazu, ein Sehnenimplantat SI mit einer Querschnittsfläche $A_S$ und ein Knochenimplantat KI mit einer Querschnittsfläche $A_K$ in einem Presssitz aufzunehmen. Besagte Querschnittsflächen $A_S$ und $A_K$ meinen die Querschnittsfläche des jeweiligen Implantats SI bzw. KI im noch nicht in der Knochenbohrung B, B' aufgenommenen bzw. verpressten Zustand; die Indizes "v" (für "verpresst") in den Fig. 2 und 5 zeigen indes an, dass dort das jeweilige Implantat SI bzw. KI im verpressten Zustand dargestellt ist, in dem die jeweilige Querschnittsfläche $A_{S_v}$ bzw. $A_{K_v}$ verformungsbedingt geringfügig kleiner sein kann als vor dem Einbringen bzw. Eintreiben der Implantate SI, KI in die Knochenbohrung B, B'. Entsprechendes gilt für einen Durchmesser $d_K$ bzw. $d_{K_v}$ des Knochenimplantats KI.

Gemäß den Fig. 1, 3 und 6 weist jedes Werkzeug 10, 12 einen Schaft 14, 14' auf, der auf gegenüberliegenden Seiten ein erstes Ende 16, 16' und ein zweites Ende 18, 18' besitzt. An dem ersten Ende 16, 16' jedes Werkzeugs 10, 12 ist ein nachfolgend noch näher beschriebener Aufweitungsabschnitt 20 bzw. 22 vorgesehen, welcher eine definierte Querschnittsfläche $A_B$ besitzt (siehe die Fig. 4 und 8) und mittels dessen die Knochenbohrung B bzw. B' in vorbestimmter Weise aufgeweitet werden kann. Während das eine Werkzeug 10 an seinem ersten Ende 16 einen für eine symmetrische Aufweitung der Knochenbohrung B ausgestalteten Aufweitungsabschnitt 20 aufweist, wie nachfolgend anhand der Fig. 2 bis 4 noch näher beschrieben wird, besitzt das andere Werkzeug 12 an seinem ersten Ende 16' einen für eine asymmetrische Aufweitung der Knochenbohrung B' ausgebildeten Aufweitungsabschnitt 22, wie im Folgenden unter Bezugnahme auf die Fig. 5 bis 9 ebenfalls noch im Detail erläutert wird. Über das verdickte zweite Ende 18, 18' jedes Werkzeugs 10, 12 kann eine äußere Kraft in das Werkzeug 10, 12 eingeleitet werden, und zwar entweder in einer Richtung vom zweiten Ende 18, 18' zum ersten Ende 16, 16', um das Werkzeug 10, 12 in die Knochenbohrung B, B' einzutreiben, oder in einer Richtung vom ersten Ende 16, 16' zum zweiten Ende 18, 18', um das Werkzeug 10, 12 aus der Knochenbohrung B, B' auszutreiben.

Wesentlich und beiden Werkzeugvarianten (10 bzw. 12) gemein ist, dass die Querschnittsfläche $A_B$ des Aufweitungsabschnitts 20 bzw. 22 (siehe die Fig. 4 bzw. 8) sich nach der folgenden, eingangs schon angegebenen Beziehung bemisst:

$$A_B = \frac{(A_S + A_K)}{(1 + \alpha)}$$

wobei $\alpha$ einen Kompressionsfaktor bezeichnet, der, wie die Erfinder herausgefunden haben, in einem Bereich von 0,30 bis 0,60, vorzugsweise in einem Bereich von 0,32 bis 0,57, mehr bevorzugt in einem Bereich von 0,38 bis 0,5 liegen und idealerweise etwa 0,44 betragen soll, um eine "Press-Fit"-Fixation mit insbesondere hoher Primärstabilität der Verbindung des Implantats (kombiniertes Sehnen- und Knochenimplantat SI, KI) im Implantatlager (aufgeweitete Knochenbohrung B bzw. B') zu erhalten.

Wie insbesondere den Fig. 1, 3 und 6 entnommen werden kann, ist das zweite Ende 18, 18' des Schafts 14, 14' zur Einleitung der axialen Austreibkräfte mit einer Querschnittserweiterung 23, 23' versehen, an der eine dem ersten Ende 16, 16' zugewandte Krafteinleitungsfläche 24, 24' ausgebildet ist, so dass Letztere gegenüber einem sich zwischen den Enden 16, 16' und 18, 18' des Schafts 14, 14' erstreckenden Mittelabschnitt 26, 26' des Schafts 14, 14' allseits radial übersteht, mithin eine Ringfläche 24, 24' bildet, wie in den Ansichten gemäß den Fig. 4, 7 und 8 besonders gut zu erkennen ist. Diese ringförmige Krafteinleitungsfläche 24, 24' ist gemäß den Fig. 1, 3 und 6 ballig ausgestaltet, wölbt sich also nach radial außen vor.

An die ballig geformte Krafteinleitungsfläche 24, 24' schließt sich stufenlos ein kurzer zylindrischer Abschnitt 28, 28' und hieran über eine Stufe ein weiterer zylindrischer Abschnitt 30, 30' kleineren Durchmessers an, bevor das zweite Ende 18, 18' des Schafts 14, 14' mit einem kugelkalottenförmigen Endabschnitt 32, 32' abschließt. Letzterer bildet eine Kraftübertragungsfläche 34, 34' zur Einleitung der äußeren Kräfte, genauer der axialen Eintreibkräfte in Richtung des ersten Endes 16, 16' des Schafts 14, 14' aus.

Zum Eintreiben des Werkzeugs 10, 12 in die Knochenbohrung B, B' ist also an der Kraftübertragungsfläche 34, 34' des zweiten Endes 18, 18' schlagend eine Kraft aufzubringen, während zum Austreiben des Werkzeugs 10, 12 aus der Knochenbohrung B, B' an der Krafteinleitungsfläche 24, 24', d.h. der Ringfläche des zweiten Endes 18, 18' schlagend eine Kraft in entgegengesetzter Richtung aufgebracht werden kann. Ein hierfür besonders geeignetes Schlagwerkzeug (nämlich eine hammerförmige Impulsmasse, nicht gezeigt), mit dem das Werkzeug 10, 12 in die Knochenbohrung B, B' eingetrieben und anschließend wieder aus der Knochenbohrung B, B' ausgetrieben werden kann, ohne dass hierzu das Schlagwerkzeug gewechselt werden müsste, wird in der zeitgleich eingereichten deutschen Patentanmeldung DE 10 2015 007 540.3 derselben Anmelder gezeigt und im Detail beschrieben, auf die hiermit bezüglich Aufbau und Funktion eines solchen Schlagwerkzeugs ausdrücklich Bezug genommen wird.

Am anderen, ersten Ende 16, 16' des Schafts 14, 14' schließt das Werkzeug 10, 12 mit einem weiteren kugelkalottenförmigen, genauer halbkugelförmigen Endabschnitt 36, 36' ab, dessen Radius hier im Wesentlichen dem Radius des Schafts 14, 14' entspricht. Ein solcher Endabschnitt 36, 36' erleichtert in vorteilhafter Weise insbesondere ein bezüglich der Knochenbohrung B, B' zentriertes Ausrichten des jeweiligen Werkzeugs 10, 12.

Gemäß den Fig. 1 und 3 folgt am ersten Ende 16 des Werkzeugs 10 dem halbkugelförmigen Endabschnitt 36 axial unmittelbar und radial stufenlos der durchmessergleiche Aufweitungsabschnitt 20, der bei dieser Werkzeugvariante für eine symmetrische Dilatation der Knochenbohrung B einen kreisförmigen Querschnitt mit einem vorbestimmten Durchmesser $d_D$ aufweist, wie am besten in Fig. 4 zu erkennen ist. Wie eingangs schon erwähnt ergibt sich der Durchmesser $d_D$ des Aufweitungsabschnitts 20 unter Berücksichtigung der obigen allgemeinen Formel für die Querschnittsfläche $A_B$ des Aufweitungsabschnitts 20 nach geeigneter Umformung aus folgender Beziehung:

$$d_D = \sqrt{d_B{}^2 + \frac{d_S^2 + d_K^2 - d_B^2(1+\alpha)}{(1+\alpha)}}$$

wobei $d_B$ einen Durchmesser der aufzuweitenden Knochenbohrung B bezeichnet (siehe die gestrichelte Linie in Fig. 2), $d_K$ einen Durchmesser des einzusetzenden Knochenimplantats KI, $d_S$ einen mittels einer herkömmlichen Sehnenlehre (nicht gezeigt) ermittelbaren äquivalenten Durchmesser des einzusetzenden Sehnenimplantats SI und $\alpha$ den vorerwähnten Kompressionsfaktor von definierter Größe.

Aufgrund der mittels des Aufweitungsabschnitts 20 bewirkten symmetrischen Aufweitung der Knochenbohrung B, bei der das angrenzende Material des Knochens KN über dem Umfang des Aufweitungsabschnitts 20 gesehen im Wesentlichen gleichmäßig radial nach außen verdrängt wird (vgl. die Fig. 2: gestrichelte Linie für das Ursprungsmaß der Knochenbohrung B, durchgezogene Linie für die symmetrisch aufgeweitete Knochenbohrung B), ist diese Art der Aufweitung besonders schonend für den Knochen KN. Um ein Maß für die Tiefe der aufgeweiteten Knochenbohrung B zu erhalten, kann das Werkzeug 10 im Bereich des Aufweitungsabschnitts 20 mit einer ggf. beschrifteten Tiefen- bzw. Längenskala 38 versehen sein.

Weitere Details zu dem Werkzeug 12 zur asymmetrischen Dilatation sind den Fig. 6 bis 9 zu entnehmen. Gemäß den Fig. 7 bis 9 ist bei diesem Ausführungsbeispiel die Querschnittform des Aufweitungsabschnitts 22 am ersten Ende 16' des Schafts 14' für die asymmetrische Dilatation ausgehend von einem bezüglich einer Mittelachse 40' des Schafts 14' zentrierten, kreisförmigen Grundquerschnitt auf einer Seite, d.h. in Fig. 6 nach rechts (bzw. Fig. 8 nach unten) erhaben ausgebildet.

Genauer gesagt besitzt der Aufweitungsabschnitt 22 im dargestellten Ausführungsbeispiel einen ovalen Querschnitt $A_B$, dessen Umfangskontur K (siehe die Fig. 7 bis 9) durch zwei Halbkreise H gleichen Durchmessers $d_B$ und zwei die Halbkreise verbindenden Geraden G gleicher Länge $e$ gebildet ist, wobei der Mittelpunkt einer der Halbkreise auf der Mittelachse 40' sitzt. Unter erneuter Berücksichtigung der obigen allgemeinen Formel für die Querschnittsfläche $A_B$ des Aufweitungsabschnitts 22 und nach geeigneter Umformung ist die Länge e an der Umfangskontur K aus folgender Beziehung ermittelbar, wie schon eingangs erwähnt:

$$e = \frac{1}{d_B}\frac{\pi}{4}\frac{[d_S^2 + d_K^2 - d_B^2(1+\alpha)]}{(1+\alpha)}$$

Dabei bezeichnet wiederum $d_B$ einen Durchmesser der aufzuweitenden Knochenbohrung B' (siehe die gestrichelte Linie in Fig. 5), $d_K$ einen Durchmesser des einzusetzenden Knochenimplantats KI, $d_S$ einen mittels der Sehnenlehre (nicht dargestellt) ermittelbaren äquivalenten Durchmesser des einzusetzenden Sehnenimplantats SI und $\alpha$ den vorbeschriebenen Kompressionsfaktor von definierter Größe.

Somit wird die Knochenbohrung B' beim Einschlagen des Werkzeugs 12 im Querschnitt gesehen asymmetrisch ausgeformt (siehe Fig. 5), um z.B. bei der eingangs beschriebenen autologen Wiederherstellung des vorderen Kreuzbandes des Kniegelenks nach der "All-Press-Fit"-Technik zu einer oder beiden Seiten der Knochenbohrung B' ein Sehnenlager auszubilden, in welches das nur begrenzt verformbare Sehnenimplantat SI ausweichen kann, wenn das Knochenimplantat KI zur Verankerung eingeschlagen wird. Hierdurch wird insbesondere ein übermäßiges Verquetschen eines im Verhältnis querschnittsgroßen Sehnenimplantats SI verhindert. Auch wird auf diese Weise - verglichen mit symmetrisch ausgebildeten "Press-Fit"-Verbindungen - bei querschnittsgrößeren Sehnenimplantaten SI die Möglichkeit der Bildung von Spalten oder Hohlräumen weiter reduziert, in denen sich Körperflüssigkeiten ansammeln könnten, was den Einheilprozess verschlechtern würde.

Wie ferner die Fig. 6 und 7 zeigen erstreckt sich zwischen dem halbkugelförmigen Endabschnitt 36' und dem Aufweitungsabschnitt 22 des Werkzeugs 12 zur asymmetrischen Dilatation ein Übergangsabschnitt 42, dessen Querschnitt sich von einem größten Querschnitt des Endabschnitts 36' zu dem Querschnitt des Aufweitungsabschnitts 22 kontinuierlich erweitert, was wiederum einem knochenmaterialschonenden Aufweiten der Knochenbohrung B' förderlich ist. Über eine geeignete Wahl der Form der Querschnittserweiterung dieses Übergangsabschnitts 42 kann auf den Fortschritt der Aufweitung und die Höhe der örtlichen Aufweitungskräfte in radialer und axialer Richtung in Abhängigkeit vom Vortrieb des Werkzeugs 12 gezielt Einfluss genommen werden.

Anhand der Fig. 1 bis 9 wurden oben zwei Werkzeuge 10, 12 - beispielhaft und stellvertretend für ggf. weitere Werkzeuge eines Werkzeugsatzes - für eine symmetrische und eine asymmetrische Dilatation von Knochenbohrungen B, B' beschrieben. Üblicherweise besitzt ein Werkzeugsatz wenigstens zwei Werkzeuge 10 bzw. 12 für eine stufenweise Dilatation einer Knochenbohrung B bzw. B' als Presssitz-Implantatlager für das kombinierte Sehnen- und Knochenimplantat SI, KI, wobei die Werkzeuge sich hinsichtlich ihres aufweitungswirksamen Querschnitts $A_B$ am Aufweitungsabschnitt 20 bzw. 22 unterscheiden.

So kann ein Werkzeugsatz eine Mehrzahl von, z.B. acht symmetrischen Dilatatoren mit gestuften Durchmessern zur Aufweitung und/oder eine Mehrzahl von, beispielsweise acht asymmetrischen Dilatatoren mit gestuften Querschnitten zur Aufweitung aufweisen.

In einem solchen Fall ist (wenigstens) das querschnittsgrößte Werkzeug zur Ausbildung der finalen Geometrie des Implantatlagers hinsichtlich seines aufweitungswirksamen Querschnitts $A_B$ am Aufweitungsabschnitt 20 bzw. 22 wie oben beschrieben zu bemessen. Es kann aber auch jedes Werkzeug eines Werkzeugsatzes für einen bestimmten Sehnenquerschnitt $A_S$ zur Ausbildung der finalen Geometrie des dazugehörigen Implantatlagers im Hinblick auf den aufweitungswirksamen Querschnitt $A_B$ am Aufweitungsabschnitt 20 bzw. 22 nach den obigen Beziehungen bemessen werden, wodurch der Werkzeugsatz universell einsetzbar ist. Bezüglich der Stufung verschiedener Werkzeuge eines Werkzeugsatzes hat es sich schließlich als vorteilhaft erwiesen, wenn jedes Werkzeug 10 bzw. 12 des Werkzeugsatzes am Aufweitungsabschnitt 20 bzw. 22 eine Umfangskontur K (vgl. die Fig. 4 und 7 bis 9) mit definierter Länge besitzt, die sich von einem Werkzeug zum nächstgrößeren Werkzeug um einen Proportionalitätsfaktor $\beta$ vergrößert, der in einem Bereich von 1,029 bis 1,039 liegt und vorzugsweise 1,035 beträgt. Mit anderen Worten gesagt ist die Länge der Umfangskontur des nächstgrößeren Werkzeugs gleich der Länge des gerade betrachteten Werkzeugs multipliziert mit dem Proportionalitätsfaktor $\beta$.

Ein Werkzeug für die Dilatation von Knochenbohrungen in der rekonstruktiven Chirurgie hat einen Schaft mit einem ersten Ende und einem zweiten Ende. Während das erste Ende einen Aufweitungsabschnitt aufweist, welcher eine definierte Querschnittsfläche besitzt und mittels dessen die Knochenbohrung aufgeweitet werden kann, ist über das zweite Ende eine Kraft in das Werkzeug einleitbar, um es als Dilatator mit seinem Aufweitungsabschnitt in die Knochenbohrung einzutreiben oder aus dieser auszutreiben. Die aufgeweitete Knochenbohrung dient dazu, als Implantatlager ein kombiniertes Sehnen- und Knochenimplantat mit bekannten Querschnittsflächen in einem Presssitz aufzunehmen. Zur Erzielung einer möglichst hohen Primärstabilität der Verbindung des Implantats im Implantatlager ist die symmetrisch oder asymmetrisch geformte Querschnittsfläche des Aufweitungsabschnitts eines Einzeldilatators oder wenigstens eines finalen Dilatators eines Werkzeugsatzes gegenüber einer Summe der nicht verpressten Querschnittsflächen der Sehnen- und Knochenimplantate unter Berücksichtigung eines Kompressionsfaktors kleiner bemessen, für den konkrete Werte angegeben werden.

BEZUGSZEICHENLISTE

| 10 | Werkzeug |
| 12 | Werkzeug |
| 14, 14' | Schaft |
| 16, 16' | erstes Ende |
| 18, 18' | zweites Ende |
| 20 | Aufweitungsabschnitt |
| 22 | Aufweitungsabschnitt |
| 23, 23' | Querschnittserweiterung |
| 24, 24' | Krafteinleitungsfläche / Ringfläche |
| 26, 26' | Mittelabschnitt |
| 28, 28' | zylindrischer Abschnitt |
| 30, 30' | zylindrischer Abschnitt |
| 32, 32' | kugelkalottenförmiger Endabschnitt |
| 34, 34' | Kraftübertragungsfläche |
| 36, 36' | kugelkalottenförmiger Endabschnitt |
| 38, 38' | Längenskala |
| 40, 40' | Mittelachse |
| 42 | Übergangsabschnitt |

| $A_B$ | Querschnittsfläche (Werkzeug) |
| $A_K$ | Querschnittsfläche (Knochenimplantat) |
| $A_S$ | Querschnittsfläche (Sehnenimplantat) |
| B, B' | Knochenbohrung |
| $d_B$ | Durchmesser (Knochenbohrung) |
| $d_D$ | Durchmesser (Werkzeug) |
| $d_K$ | Durchmesser (Knochenimplantat) |
| $d_S$ | äquivalenter Durchmesser (Sehnenimplantat) |
| $e$ | Länge (Gerade) |
| G | Gerade |
| H | Halbkreis |
| K | Umfangskontur |
| KI | Knochenimplantat |
| KN | Knochen |
| SI | Sehnenimplantat |

| $\alpha$ | Kompressionsfaktor |
| $\beta$ | Proportionalitätsfaktor |

**Patentansprüche**

1. Kombination aus einem Werkzeug (10, 12) für die Dilatation von Knochenbohrungen (B, B') in der rekonstruktiven Chirurgie, einem Sehnenimplantat (SI) mit einer Querschnittsfläche ($A_S$) und einem Knochenimplantat (KI) mit einer Querschnittsfläche ($A_K$), wobei das Werkzeug (10, 12) einen Schaft (14, 14') aufweist, der ein erstes Ende (16, 16') mit einem Aufweitungsabschnitt (20, 22) hat, welcher eine definierte Querschnittsfläche ($A_B$) besitzt und mittels dessen die Knochenbohrung (B, B') aufweitbar ist, wobei der Schaft (14, 14') ferner ein zweites Ende (18, 18') aufweist, über das eine Kraft einleitbar ist, um das Werkzeug (10, 12) in die Knochenbohrung (B, B') einzutreiben oder aus dieser auszutreiben, wobei die Knochenbohrung (B, B') dazu dient, das Sehnenimplantat (SI) und das Knochenimplantat (KI) in einem Presssitz aufzunehmen, **dadurch gekennzeichnet, dass** die Querschnittsfläche ($A_B$) des Aufweitungsabschnitts (20, 22) sich nach folgender Beziehung bemisst:

$$A_B = \frac{(A_S + A_K)}{(1 + \alpha)}$$

mit $\alpha$ : einem Kompressionsfaktor, der in einem Bereich von 0,30 bis 0,60 liegt.

2. Kombination nach Anspruch 1, **dadurch gekennzeichnet, dass** der Aufweitungsabschnitt (20) des ersten Endes (16) des Schafts (14) für eine symmetrische Dilatation der Knochenbohrung (B) einen kreisförmigen Querschnitt mit einem Durchmesser ($d_D$) aufweist.

3. Kombination nach Anspruch 2, **dadurch gekennzeichnet, dass** der Durchmesser ($d_D$) des Aufweitungsabschnitts (20) nach folgender Beziehung ermittelbar ist:

$$d_D = \sqrt{d_B{}^2 + \frac{d_S^2 + d_K^2 - d_B^2(1 + \alpha)}{(1 + \alpha)}}$$

mit

$d_B$: einem Durchmesser der Knochenbohrung (B),

$d_K$: einem Durchmesser des Knochenimplantats (KI),

$d_S$: einem äquivalenten Durchmesser des Sehnenimplantats (SI) und

$\alpha$: dem vorerwähnten Kompressionsfaktor.

4. Kombination nach Anspruch 1, **dadurch gekennzeichnet, dass** eine Querschnittform des Aufweitungsabschnitts (22) des ersten Endes (16') des Schafts (14') für eine asymmetrische Dilatation ausgehend von einem bezüglich einer Mittelachse (40') des Schafts (14') zentrierten, kreisförmigen Grundquerschnitt auf einer Seite oder auf bezüglich der Mittelachse (40') diametral gegenüberliegenden Seiten erhaben ausgebildet ist.

5. Kombination nach Anspruch 4, **dadurch gekennzeichnet, dass** der Aufweitungsabschnitt (22) des ersten Endes (16') des Schafts (14') einen ovalen Querschnitt ($A_B$) besitzt, dessen Umfangskontur (K) durch zwei Halbkreise (H) gleichen Durchmessers ($d_B$) und zwei die Halbkreise (H) verbindenden Geraden (G) gleicher Länge ($e$) gebildet ist, wobei die Länge ($e$) nach der folgenden Beziehung ermittelbar ist:

$$e = \frac{1}{d_B} \frac{\pi}{4} \frac{[d_S^2 + d_K^2 - d_B^2(1 + \alpha)]}{(1 + \alpha)}$$

mit

$d_B$: einem Durchmesser der Knochenbohrung (B'),

$d_K$: einem Durchmesser des Knochenimplantats (KI),

$d_S$: einem äquivalenten Durchmesser des Sehnenimplantats (SI) und

$\alpha'$: dem vorerwähnten Kompressionsfaktor.

6. Kombination nach Anspruch 4 oder 5, **dadurch gekennzeichnet, dass** sich zwischen einem Endabschnitt (36') und dem Aufweitungsabschnitt (22) des ersten Endes (16') des Schafts (14') ein Übergangsabschnitt (42) erstreckt, dessen Querschnitt sich von einem größten Querschnitt des Endabschnitts (36') zu dem Querschnitt ($A_B$) des Aufweitungsabschnitts (22) kontinuierlich erweitert.

7. Kombination nach einem der vorhergehenden Ansprüche, **dadurch gekennzeichnet, dass** das erste Ende (16, 16') des Schafts (14, 14') einen kugelkalottenförmigen Endabschnitt (36, 36') aufweist.

8. Kombination nach einem der vorhergehenden Ansprüche, **dadurch gekennzeichnet, dass** das zweite Ende (18, 18') des Schafts (14, 14') zur Einleitung axialer Austreibkräfte mit einer Querschnittserweiterung (23, 23') versehen ist.

9. Kombination nach Anspruch 8, **dadurch gekennzeichnet, dass** die Querschnittserweiterung (23, 23') auf ihrer dem ersten Ende (16, 16') des Schafts (14, 14') zugewandten Seite eine ballig ausgestaltete Ringfläche (24, 24') aufweist.

10. Kombination nach einem der vorhergehenden Ansprüche, **dadurch gekennzeichnet, dass** das zweite Ende (18, 18') des Schafts (14, 14') zur Einleitung axialer Eintreibkräfte einen kugelkalottenförmigen Endabschnitt (32, 32') aufweist.

11. Kombination aus einem Werkzeugsatz mit wenigstens zwei Werkzeugen (10, 12) für eine stufenweise Dilatation einer Knochenbohrung (B, B') als Presssitz-Implantatlager für ein kombiniertes Sehnen- und Knochenimplantat (SI, KI), einem Sehnenimplantat (SI) und einem Knochenimplantat (KI), wobei die Werkzeuge sich hinsichtlich ihres aufweitungswirksamen Querschnitts unterscheiden und das querschnittsgrößte Werkzeug zur Ausbildung der finalen Geometrie des Implantatlagers gemäß einem der vorhergehenden Ansprüche ausgebildet ist.

12. Kombination aus einem Werkzeugsatz mit wenigstens zwei Werkzeugen (10, 12) für eine stufenweise Dilatation einer Knochenbohrung (B, B') als Presssitz-Implantatlager für ein kombiniertes Sehnen- und Knochenimplantat (SI, KI), einem Sehnenimplantat (SI) und einem Knochenimplantat (KI), wobei die Werkzeuge sich hinsichtlich ihres aufweitungswirksamen Querschnitts unterscheiden und so im Querschnitt gestuft sind, dass jedes Werkzeug für einen bestimmten Sehnenquerschnitt ($A_S$) zur Ausbildung der finalen Geometrie des dazugehörigen Implantatlagers gemäß einem der Ansprüche 1 bis 10 ausgebildet ist.

13. Kombination nach Anspruch 11 oder 12, **dadurch gekennzeichnet, dass** jedes Werkzeug (10, 12) am Aufweitungsabschnitt (20, 22) eine Umfangskontur (K) mit definierter Länge besitzt, die sich von einem Werkzeug zum nächstgrößeren Werkzeug um einen Proportionalitätsfaktor ($\beta$) vergrößert, der in einem Bereich von 1,029 bis 1,039 liegt und vorzugsweise 1,035 beträgt.

14. Kombination aus einem Werkzeug für die Erzeugung einer Aussparung in einem Knochen in der rekonstruktiven Chirurgie, einem Sehnenimplantat mit einer Querschnittsfläche ($A_S$) und einem Knochenimplantat mit einer Querschnittsfläche ($A_K$), mit einem ersten Werkzeugteil, welcher eine definierte Querschnittsfläche ($A_B$) besitzt und dessen Geometrie die Geometrie der erzeugten Aussparung im Knochen bestimmt, so dass die Querschnittsfläche der erzeugten Aussparung im Knochen der Querschnittsfläche ($A_B$) des ersten Werkzeugteils entspricht, und einem zweiten Werkzeugteil, über den das Werkzeug zur Erzeugung der Aussparung relativ zum Knochen zustellbar ist, wobei die Aussparung im Knochen dazu dient, das Sehnenimplantat und das Knochenimplantat in einem Presssitz aufzunehmen, und wobei die Querschnittsfläche ($A_B$) des ersten Werkzeugteils sich nach folgender Beziehung bemisst:

$$A_B = \frac{(A_S + A_K)}{(1 + \alpha)}$$

mit $\alpha$ : einem Kompressionsfaktor, der in einem Bereich von 0,30 bis 0,60 liegt.

15. Kombination nach einem der Ansprüche 1 bis 10 oder Anspruch 14, **dadurch gekennzeichnet, dass** der Kompressionsfaktor ($\alpha$) in einem Bereich von 0,32 bis 0,57 oder in einem Bereich von 0,38 bis 0,5 liegt oder etwa 0,44 beträgt.

**Claims**

1. Combination of a tool (10, 12) for the dilatation of drilled bone holes (B, B') in reconstructive surgery, a tendon implant (SI) with a cross-sectional area ($A_S$) and a bone implant (KI) with a cross-sectional area ($A_K$), wherein the tool (10, 12) comprises a shank (14, 14') having a first end (16, 16') with an expansion section (20, 22), which has a defined cross-sectional area ($A_B$) and by means of which the drilled bone hole (B, B') can be expanded, wherein the shank (14, 14') further has a second end (18, 18') by way of which a force can be introduced in order to drive the tool (10, 12) into or drive the tool out of the drilled bone hole (B, B'), wherein the drilled bone hole (B, B') serves the purpose of receiving the tendon implant (SI) and the bone implant (KI) in a press fit, **characterized in that** the cross-sectional area ($A_B$) of the expansion section (20, 22) is dimensioned in accordance with the following equation:

$$A_B = \frac{(A_S + A_K)}{(1 + \alpha)}$$

wherein $\alpha$ is a compression factor lying in the range of 0.30 to 0.60.

2. Combination according to claim 1, **characterized in that** the expansion section (20) of the first end (16) of the shank (14) has, for symmetrical dilatation of the drilled bone hole (B), a circular cross-section with a diameter ($d_D$).

3. Combination according to claim 2, **characterized in that** the diameter ($d_D$) of the expansion section (20) is determinable in accordance with the following equation:

$$d_D = \sqrt{d_B{}^2 + \frac{d_S^2 + d_K^2 - d_B^2(1 + \alpha)}{(1 + \alpha)}}$$

wherein

$d_B$: is a diameter of the drilled bone hole (B),
$d_K$: is a diameter of the bone implant (KI),
$d_S$: is an equivalent diameter of the tendon implant (SI) and
$\alpha$: is the afore-mentioned compression factor.

4. Combination according to claim 1, **characterized in that** a cross-sectional shape of the expansion section (22) of the first end (16') of the shank (14') is, for asymmetrical dilatation, constructed to be elevated starting from a circular basic cross-section, which is centered with respect to a center axis (40') of the shank (14'), to one side or to sides which are diametrically opposite with respect to the center axis (40').

5. Combination according to claim 4, **characterized in that** the expansion section (22) of the first end (16') of the shank (14') has an oval cross-section ($A_B$), the circumferential profile (K) of which is defined by two semicircles (H) of the same diameter ($d_B$) and two straight lines (G) of equal length (e) connecting the semicircles, wherein the length (e) is determinable in accordance with the following equation:

$$e = \frac{1}{d_B}\frac{\pi}{4}\frac{[d_S^2 + d_K^2 - d_B^2(1 + \alpha)]}{(1 + \alpha)}$$

wherein

$d_B$: is a diameter of the drilled bone hole (B'),
$d_K$: is a diameter of the bone implant (KI),
$d_S$: is an equivalent diameter of the tendon implant (SI) and
$\alpha$: is the afore-mentioned compression factor.

6. Combination according to claim 4 or 5, **characterized in that** a transition section (42) extends between an end section (36') and the expansion section (22) of the first end (16') of the shank (14'), and has a cross-section continuously widening from a largest cross-section of the end section (36') to the cross-section ($A_B$) of the expansion section (22).

7. Combination according to any one of the preceding claims, **characterized in that** the first end (16, 16') of the shank (14, 14') has an end section (36, 36') of spherical calotte shape.

8. Combination according to any one of the preceding claims, **characterized in that** the second end (18, 18') of the shank (14, 14') is provided with a cross-sectional widening (23, 23') for introduction of axial driving-out forces.

9. Combination according to claim 8, **characterized in that** the cross-sectional widening (23, 23') has a bulged annular surface (24, 24') on the side thereof facing the first end (16, 16') of the shank (14, 14').

10. Combination according to any one of the preceding claims, **characterized in that** the second end (18, 18') of the shank (14, 14') has an end section (32, 32') of spherical calotte shape for introduction of axial driving-in forces.

11. Combination of a tool set with at least two tools (10, 12) for stepped dilatation of a drilled bone hole (B, B') as a press-fit implant bed for a combined tendon and bone implant (SI, KI), a tendon implant (SI) and a bone implant (KI), wherein the tools differ with respect to the cross-section thereof effective in terms of expansion and the cross-sectionally largest tool is constructed for realization of the final geometry of the implant bed, according to any one of the preceding claims.

12. Combination of a tool set with at least two tools (10, 12) for stepped dilatation of a drilled bone hole (B, B') as a press-fit implant bed for a combined tendon and bone implant (SI, KI), a tendon implant (SI) and a bone implant (KI), wherein the tools differ with respect to the cross-section thereof effective in terms of expansion and are so stepped in cross-section that each tool is constructed for a specific tendon cross-section (As) for realization of the final geometry of the associated implant bed, according to any one of the claims 1 to 10.

13. Combination according to claim 11 or 12, **characterized in that** each tool (10, 12) has at the expansion section (20, 22) a circumferential profile (K) with defined length which increases from one tool to the next larger tool by a proportionality factor ($\beta$) lying in a range of 1.029 to 1.039 and preferably amounting to 1.035.

14. Combination of a tool for producing a recess in a bone in reconstructive surgery, a tendon implant with a cross-sectional area ($A_S$) and a bone implant with a cross-sectional area ($A_K$), with a first tool part which has a defined cross-sectional area ($A_B$) and the geometry of which determines the geometry of the recess produced in the bone, so that the cross-sectional area of the recess produced in the bone corresponds with the cross-sectional area ($A_B$) of the first tool part, and a second tool part by way of which the tool for producing the recess is infeedable relative to the bone, wherein the recess in the bone serves the purpose of receiving the tendon implant and the bone implant in a press fit, and wherein the cross-sectional area ($A_B$) of the first tool part is dimensioned in accordance with the following equation:

$$A_B = \frac{(A_S + A_K)}{(1 + \alpha)}$$

wherein $\alpha$ : is a compression factor, which lies in a range of 0.30 to 0.60.

15. Combination according to any one of claims 1 to 10 or claim 14, **characterized in that** the compression factor ($\alpha$) lies in a range of 0.32 to 0.57 or in a range of 0.38 to 0.5 or is approximately 0.44.

## Revendications

1. Combinaison d'un outil (10, 12) pour la dilatation de trous dans l'os (B, B') dans la chirurgie reconstructive, d'un implant tendineux (SI) avec une surface de section transversale ($A_S$) et d'un implant osseux (KI) avec une surface de section transversale ($A_K$), dans laquelle l'outil (10, 12) présente une tige (14, 14'), qui a une première extrémité (16, 16') avec une partie d'élargissement (20, 22), laquelle possède une surface de section transversale ($A_B$) définie et au moyen de laquelle le trou dans l'os (B, B') peut être élargi, dans laquelle la tige (14, 14') présente en outre une deuxième extrémité (18, 18'), par l'intermédiaire de laquelle une force peut être appliquée, afin d'enfoncer l'outil (10, 12) dans le trou dans l'os (B, B') ou de l'expulser de celui-ci, dans laquelle le trou dans l'os (B, B') sert à recevoir l'implant tendineux (SI) et l'implant osseux (KI) dans un ajustement serré, **caractérisée en ce que** la surface de section transversale ($A_B$) de la partie d'élargissement (20, 22) se mesure selon la relation suivante :

$$A_B = \frac{(A_S + A_K)}{(1 + \alpha)}$$

avec $\alpha$ : un facteur de compression qui se situe dans une plage de 0,30 à 0,60.

2. Combinaison selon la revendication 1, **caractérisée en ce que** la partie d'élargissement (20) de la première extrémité (16) de la tige (14) présente pour une dilatation symétrique du trou dans l'os (B) une section transversale circulaire avec un diamètre ($d_D$).

3. Combinaison selon la revendication 2, **caractérisée en ce que** le diamètre ($d_D$) de la partie d'élargissement (20) peut être déterminé selon la relation suivante :

$$d_D = \sqrt{d_B{}^2 + \frac{d_S^2 + d_K^2 - d_B^2(1 + \alpha)}{(1 + \alpha)}}$$

avec $d_B$ : un diamètre du trou dans l'os (B),
$d_K$ : un diamètre de l'implant osseux (KI),
$d_S$ : un diamètre équivalent de l'implant tendineux (SI) et
$\alpha$ : le facteur de compression précédemment mentionné.

4. Combinaison selon la revendication 1, **caractérisée en ce qu'**une forme de section transversale de la partie d'élargissement (22) de la première extrémité (16') de la tige (14') pour une dilatation asymétrique est réalisée à partir d'une section transversale de base circulaire, centrée par rapport à un axe médian (40') de la tige (14'), sur une face ou sur des faces diamétralement opposées par rapport à l'axe médian (40').

5. Combinaison selon la revendication 4, **caractérisée en ce que** la partie d'élargissement (22) de la première extrémité (16') de la tige (14') possède une section transversale ($A_B$) ovale, dont le contour périphérique (K) est formé par deux demi-cercles (H) de diamètre ($d_B$) identique et deux droites (G) de longueur ($e$) identique reliant les demi-cercles (H), dans laquelle la longueur ($e$) peut être déterminée selon la relation suivante :

$$e = \frac{1}{d_B} \frac{\pi}{4} \frac{[d_S^2 + d_K^2 - d_B^2(1 + \alpha)]}{(1 + \alpha)}$$

avec $d_B$ : un diamètre du trou dans l'os (B'),
$d_K$ : un diamètre de l'implant osseux (KI),
$d_S$ : un diamètre équivalent de l'implant tendineux (SI) et
$\alpha$ : le facteur de compression précédemment mentionné.

6. Combinaison selon la revendication 4 ou 5, **caractérisée en ce qu'**une partie de transition (42), dont la section transversale s'élargit en continu d'une plus grande section transversale de la partie d'extrémité (36') à la section transversale ($A_B$) de la partie d'élargissement (22), s'étend entre une partie d'extrémité (36') et la partie d'élargissement (22) de la première extrémité (16') de la tige (14').

7. Combinaison selon l'une quelconque des revendications précédentes, **caractérisée en ce que** la première extrémité (16, 16') de la tige (14, 14') présente une partie d'extrémité (36, 36') en forme de calotte sphérique.

8. Combinaison selon l'une quelconque des revendications précédentes, **caractérisée en ce que** la deuxième extrémité (18, 18') de la tige (14, 14'), pour l'application de forces d'expulsion axiales, est pourvue d'un élargissement de section transversale (23, 23').

9. Combinaison selon la revendication 8, **caractérisée en ce que** l'élargissement de section transversale (23, 23') présente sur sa face tournée vers la première extrémité (16, 16') de la tige (14, 14') une surface annulaire (24, 24') bombée.

10. Combinaison selon l'une quelconque des revendications précédentes, **caractérisée en ce que** la deuxième extrémité (18, 18') de la tige (14, 14'), pour l'application de forces d'enfoncement axiales, présente une partie d'extrémité (32, 32') en forme de calotte sphérique.

11. Combinaison d'un jeu d'outils avec au moins deux outils (10, 12) pour une dilatation graduelle d'un trou dans l'os (B, B') en tant que lit implantaire à ajustement serré pour un implant tendineux et osseux combiné (SI, KI), un implant tendineux (SI) et un implant osseux (KI), dans laquelle les outils se différencient par leur section transversale agissant sur l'élargissement et l'outil à plus grande section transversale est conçu pour réaliser la géométrie finale du lit implantaire selon l'une quelconque des revendications précédentes.

12. Combinaison d'un jeu d'outils avec au moins deux outils (10, 12) pour une dilatation graduelle d'un trou dans l'os (B, B') en tant que lit implantaire à ajustement serré pour un implant tendineux et osseux combiné (SI, KI), un implant tendineux (SI) et un implant osseux (KI), dans laquelle les outils se différencient par leur section transversale agissant sur l'élargissement et sont classés par section transversale, de sorte que chaque outil pour une section transversale de tendon ($A_S$) définie est conçu pour réaliser la géométrie finale du lit implantaire associé selon l'une des revendications 1 à 10.

13. Combinaison selon la revendication 11 ou 12, **caractérisée en ce que** chaque outil (10, 12) possède sur la partie d'élargissement (20, 22) un contour périphérique (K) de longueur définie, qui augmente d'un outil à l'outil immédiatement plus grand d'un facteur de proportionnalité ($\beta$), qui se situe dans une plage de 1,029 à 1,039 et atteint de préférence 1,035.

14. Combinaison d'un outil pour la production d'un évidement dans un os dans la chirurgie reconstructive, d'un implant tendineux avec une surface de section transversale ($A_S$) et d'un implant osseux avec une surface de section transversale ($A_K$), avec une première partie d'outil, laquelle possède une surface de section transversale ($A_B$) définie et dont la géométrie définit la géométrie de l'évidement produit dans l'os, de sorte que la surface de section transversale de l'évidement produit dans l'os correspond à la surface de section transversale ($A_B$) de la première partie d'outil, et une deuxième partie d'outil, par l'intermédiaire de laquelle l'outil destiné à produire l'évidement peut être avancé par rapport à l'os, dans laquelle l'évidement dans l'os sert à recevoir l'implant tendineux et l'implant osseux dans un ajustement serré, et dans laquelle la surface de section transversale ($A_B$) de la première partie d'outil se mesure selon la relation suivante :

$$A_B = \frac{(A_S + A_K)}{(1 + \propto)}$$

avec $\alpha$ : un facteur de compression qui se situe dans une plage de 0,30 à 0,60.

15. Combinaison selon l'une quelconque des revendications 1 à 10 ou selon la revendication 14, **caractérisée en ce que** le facteur de compression ($\alpha$) se situe dans une plage de 0,32 à 0,57 ou dans une plage de 0,38 à 0,5 ou atteint sensiblement 0,44.

FIG. 1

16

## FIG. 3

*20* · *36* · *16*

10 · 15 · 20 · 25 · 30 · 35 · 40

*IV* · *IV*

*38*

*40*

*14*

*10*

*26*

*24* · *18*

*23* · *28*

*30*

*34* · *32*

## FIG. 2

$d_{Kv}$

$A_{Sv}$

$B$

$SI$

$d_B$

$A_{Kv}$

$Kl$

$KN$

## FIG. 4

*24* · *10* · $A_B$

$d_D$

*40* · *20*

*K*

**FIG. 5**

**FIG. 6**

**FIG. 7**

**FIG. 8**

**FIG. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202014007804 U1
- US 2010249930 A1

- DE 102015007540